Europäisches Patentamt

European Patent Office

Office européen des brevets

(19)

(11) Veröffentlichungsnummer: **0 229 872**
**B1**

(12) ## EUROPÄISCHE PATENTSCHRIFT

(45) Veröffentlichungstag der Patentschrift:
16.08.89

(51) Int. Cl.⁴: **C12M 1/12**, B01D 13/00

(21) Anmeldenummer: **86111855.2**

(22) Anmeldetag: **27.08.86**

(54) Membrantrennverfahren und Vorrichting zur Abtrennung von Flüssigkeiten aus Fermentationssuspensionen.

(30) Priorität: **06.09.85 DE 3531836**

(43) Veröffentlichungstag der Anmeldung:
**29.07.87 Patentblatt 87/31**

(45) Bekanntmachung des Hinweises auf die Patenterteilung:
**16.08.89 Patentblatt 89/33**

(84) Benannte Vertragsstaaten:
**AT FR GB IT NL SE**

(56) Entgegenhaltungen:
**EP-A- 0 086 539**
**AU-A- 415 381**
**FR-A- 2 430 451**
**GB-A- 1 238 401**

(73) Patentinhaber: **Starcosa GmbH, Am Alten Bahnhof 5,
D-3300 Braunschweig(DE)**

(72) Erfinder: **Storkebaum, Christoph, Wilhelmitorwall 33a,
D-3300 Braunschweig(DE)**
Erfinder: **Tegtmeier, Uwe, Dr., Eichenweg 5,
D-3341 Wittmar(DE)**

(74) Vertreter: **Döring, Rudolf, Dr.-Ing., Patentanwälte
Dr.-Ing. R. Döring Dipl.-Phys. Dr. J. Fricke Jasperallee 1a,
D-3300 Braunschweig(DE)**

## Beschreibung

Die Erfindung betrifft ein Verfahren zur Abtrennung von Flüssigkeiten aus Fermentationssuspensionen gemäß dem Gattungsbegriff des Anspruches 1 sowie eine Vorrichtung zur Durchführung des Verfahrens.

Es ist ein Verfahren der vorgenannten Art bekannt (GB-PS 1 209 769).

Bei der Druckfiltration nach dem Stand der Technik verursacht der angewendete hohe Druck und insbesondere die nach der Filtration erforderliche Entspannung des Retentats eine erhebliche Schädigung bestimmter Mikroorganismen.

Der Erfindung liegt die Aufgabe zugrunde, ein Verfahren der einleitend genannten Art so weiterzubilden, daß bei geringerem Energieaufwand für die Erzeugung des Druckgefälles eine schonende Behandlung des Retentates bei der Trennung druckempfindlicher Substanzen erzielt und eine sterile Prozeßführung ermöglicht werden.

Zur Lösung vorstehender Aufgabe kennzeichnet sich das einleitend genannte Verfahren erfindungsgemäß dadurch, daß auf der Permeatseite der Membran ein Unterdruck erzeugt wird, indem das Permeat mittels einer Strahlpumpe in flüssigem Zustand abgesaugt und in eine Kreislaufströmung überführt sowie innerhalb der Kreislaufströmung als Treibmittel der Strahlpumpe zugeleitet wird.

Dadurch, daß auf der Permeatseite der Membran ein Unterdruck erzeugt wird, kann die Zuführung der Suspension in den Zulauf- bzw. Retentatraum mit relativ geringem Druck erfolgen, da das für die Trennung notwendige Druckgefälle ganz oder zumindest zum erheblichen Teil durch die Erzeugung des Unterdruckes auf der Permeatseite der Membran erreicht wird. Zwar wird für die Erzeugung dieses Unterdruckes und die Kreislaufführung des Permeats ebenfalls Energie benötigt, jedoch ist die Summe der erforderlichen Energie für die Erzeugung des geringen Überdruckes in dem Zulaufbzw. Retentatraum und des Unterdruckes auf der Permeatseite der Membran wesentlich geringer als bei herkömmlichen Verfahren für die Erzeugung des relativ hohen Überdruckes in dem Zulauf- bzw. Retentatraum, wenn auf der Permeatseite mit Atmosphärendruck gearbeitet wird.

Durch die geringeren Druckdifferenzen zwischen der Zulauf- bzw. Retentatseite und der Atmosphäre erfolgt eine entsprechend geringere Druckbeanspruchung des Retentates, so daß auch relativ scherempfindliche Zellen, wie tierische Zellen oder submers wachsende Pilzkulturen, nicht durch die Druckbeanspruchung nachteilig beeinträchtigt bzw. beschädigt werden.

Durch die Erzeugung des Unterdruckes auf der Permeatseite der Membran einerseits und des Überdruckes auf der Zulauf- bzw. Retentatseite der Membran andererseits erreicht man außerdem eine gleichmäßigere Druckverteilung entlang der Membran, so daß auch eine gleichmäßigere Belastung der Membran und damit eine höhere Ausbeute erreicht wird.

Da für die Absaugung des Permeats von der Permeatseite der Membran das bereits zuvor dem Prozeß entnommene Permeat als Treibstrahl dient, ermöglicht das neue Verfahren auch eine sterile Prozeßführung.

Durch die Absaugung der Permeatseite der Membran mittels der Strahlpumpe werden gegenüber der möglichen Verwendung von Vakuumpumpen erhebliche Vorteile dadurch erzielt, daß unerwünschte Verdampfungen vermieden werden oder, wenn diese doch auftreten sollten, ohne zusätzliche Maßnahmen wieder in die Flüssigphase zurückgeführt werden.

Der maximal erreichbare Unterdruck auf der Permeatseite mittels der Strahlpumpe richtet sich entsprechend dem Dampfdruck nach der Temperatur des als Treibmittel in der Kreislaufströmung wirkenden Permeats. Aus diesem Grunde ist es zweckmäßig, wenn das Permeat während der Kreislaufströmung gekühlt wird.

Zur Durchführung des Verfahrens geht die Erfindung von bekannten Vorrichtungen aus, die mit einer Membranfilteranordnung sowie mit Einrichtungen für den Zulauf der Suspension und für den Retentatablauf auf der einen Seite sowie Einrichtungen für den Permeatablauf auf der anderen Seite der Membran ausgerüstet sind und einen mit der Permeatablaufleitung verbundenen Sammelbehälter aufweisen, der mit einer Abführleitung und einer Niveausteuerung für das abzuführende Permeat ausgerüstet ist. Erfindungsgemäß kennzeichnet sich diese Vorrichtung dadurch, daß der Sammelbehälter im Zuge einer Kreislaufleitung in Reihe mit Förderpumpe und einer Strahlpumpe angeordnet ist, daß die von der Filteranordnung ausgehende Permeatablaufleitung mit der Strahlpumpe verbunden ist, und daß zwischen der Förderpumpe und der Strahlpumpe ein Dreiwegeventil mit einer in die Permeatablaufleitung mündenden Rückspülleitung vorgesehen und die Retentatablaufleitung über ein Dreiwegeventil mit dem Fermenter für die Suspension verbindbar ist.

Auf diese Weise kann allein mit Hilfe der Förderpumpe die Rückspülung der Filteranordnung mittels des zuvor gewonnenen Permeates erfolgen.

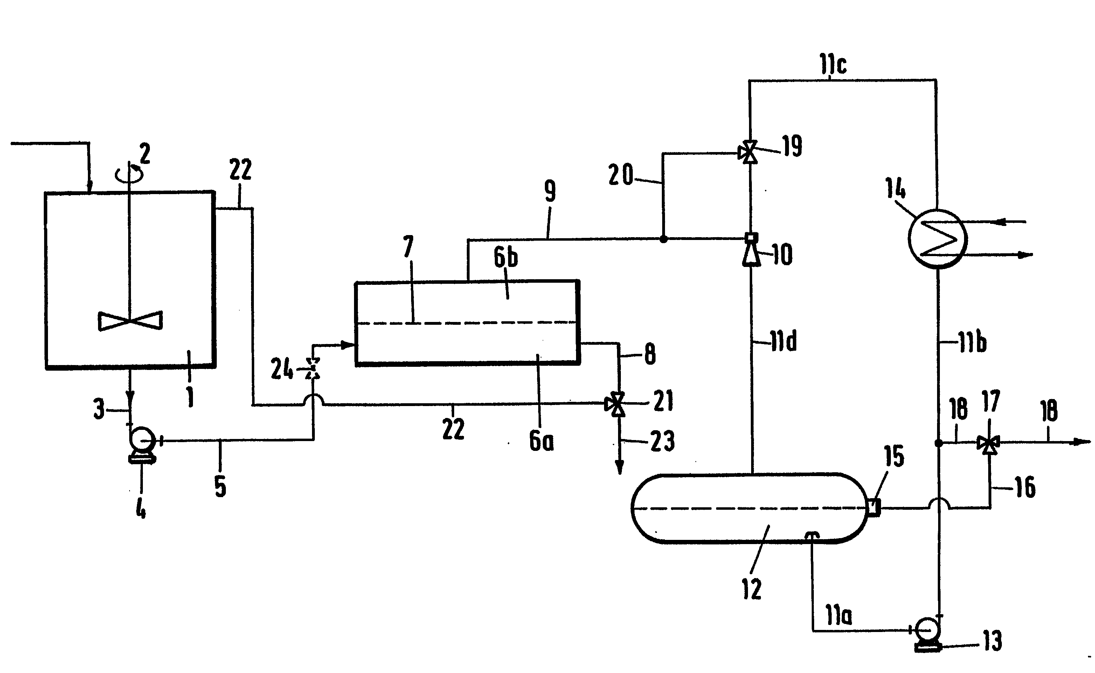

Anhand der Zeichnung wird das Verfahren näher erläutert.

Die Zeichnung gibt in rein schematischer Darstellung ein Ausführungsbeispiel für eine Vorrichtung zur Durchführung des Verfahrens nach der Erfindung wieder.

In der Zeichnung ist mit 1 ein Fermenter bezeichnet, welcher mit einem Rührwerk 2 ausgerüstet ist und dessen Austrittsleitung 3 mit einer Förderpumpe 4 verbunden ist, welche über die Druckleitung 5 die aus dem Fermenter ausströmende Suspension in die insgesamt mit 6 bezeichnete Membranfilteranordnung pumpt. Die Membranfilteranordnung 6 ist durch die Membran 7 in einen Zulauf- bzw. Retentatraum 6a und einen Permeatraum 6b unterteilt. Die Zulaufleitung 5 mündet in den Zulauf- bzw. Retentatraum 6a aus. Ihr gegenüberliegend ist die Retentatablaufleitung 8 vorgesehen. Das Retentat kann wahlweise mittels eines Dreiwegeventiles 21 über Leitung 22 zum Fermenter 1 zurückgeführt oder aus dem Prozeß abgeleitet werden. Der Permeatraum 6b ist über eine Permetablaufleitung 9 mit einer Strahlpumpe 10 verbunden. Diese Strahlpumpe 10 ist im Zuge einer Kreislaufleitung 11a bis 11d angeordnet. Die Kreislaufleitung 11a bis 11d erstreckt sich von der Unterseite eines Permeatsammelbehälters 12 über einen Leitungsabschnitt 11a zur Saugseite einer Förderpumpe 13 und von der Druckseite der Förderpumpe 13 über einen weiteren Leitungsabschnitt 11b zu einer als Wärmetauscher ausgebildeten Kühleinrichtung 14 und von dort weiter über einen Leitungsabschnitt 11c bis zur Strahlpumpe 10 und von dort aus über den Leitungsabschnitt 11d bis zur Oberseite des Permeatsammelbehälters 12.

Der Permeatsammelbehälter 12 ist über eine Niveausteuereinrichtung 15 mit einer Permeatabführungsleitung 16 verbunden, welche über ein Dreiwegeventil 17 mit einer Leitung 18 zur Weiterförderung des Permeats verbunden ist. Über das Ventil 17 kann die Weiterförderungsleitung 18 auch unmittelbar mit dem Leitungsabschnitt 11b der Kreislaufleitung verbunden werden.

Beim Betrieb der beschriebenen Vorrichtung wird die aus dem Fermenter 1 über die Pumpe 4 abgezogene Suspension über die Leitung 5 der Membranfilteranordnung 6 zugeführt. Dabei baut sich in dem Zulauf- bzw. Retentatraum 6a ein Druck auf, welcher durch den Pumpendruck erzeugt wird und mittels nicht in der Zeichnung wiedergegebener Steuereinrichtungen auf unterschiedliche Werte eingestellt werden kann.

Beim Betrieb der Anordnung erfolgt eine Förderung des bereits zuvor gewonnenen Permeats durch die Kreislaufleitung 11a bis 11d mittels der Pumpe 13, wobei das im Kreislauf geförderte Permeat als Treibmittel für die Strahlpumpe 10 wirksam ist. Durch die Verbindung der Strahlpumpe 10 über die Permeatablaufleitung 9 mit dem Permeatraum 6b der Membranfilteranordnung 6 wird in dem Permeatraum 6b ein Unterdruck erzeugt, welcher sich zusammen mit dem auf der Zulauf- bzw. Retentatseite durch die Förderpumpe 4 erzeugten Überdruck als Trenndruck auswirkt.

Mittels der Strahlpumpe 10 wird das Permeat aus dem Permeatraum 6b in die Kreislaufleitung 11a bis 11d gefördert und gelangt über den Leitungsabschnitt 11d in den Permeatsammelbehälter 12. Aus diesem wird niveaugesteuert jeweils ein Teil des im Kreislauf geführten Permeats über die Permeatabführungsleitung in die Weiterförderungsleitung 18 abgeführt.

Durch die Erzeugung des Unterdruckes in dem Permeatraum 6b der Membranfilteranordnung 6 kann der in dem Zulauf- bzw. Retentatraum 6a erforderliche Überdruck entsprechend geringer gehalten werden, so daß eine schonende Behandlung druck- bzw. scherkraftempfindlicher Substanzen erreicht und der Energieaufwand für die Erzielung der gesamten Druck differenz in der Membranfilteranordnung 6 erheblich gegenüber einem Betrieb mit atmosphärischem Druck in dem Permeatraum 6b vermindert werden kann.

Zum Zwecke der Rückspülung der Membranfilteranordnung ist im Abschnitt 11c der Kreislaufleitung 11a bis 11d ein Dreiwegeventil 19 vorgesehen, welches über eine Rückspülleitung 20 mit der von der Membranfilteranordnung 6 ausgehenden Permeatablaufleitung 9 verbunden ist. Weiterhin ist für die Rückspülung in der Retentatablaufleitung 8 ebenfalls ein Dreiwegeventil 21 vorgesehen, welches es erlaubt, wahlweise eine Rückführung des Spülmediums zum Fermenter 1 oder eine Ableitung 23 aus dem Prozeß vorzunehmen.

Zur Rückspülung wird das Dreiwegeventil 19 in dem Leitungsabschnitt 11c so eingestellt, daß es den Zufluß zur Strahlpumpe 10 sperrt und den Leitungsabschnitt 11c mit der Rückspülleitung 20 verbindet. Das Dreiwegeventil 21 in der Retentatablaufleitung 8 wird üblicherweise so eingestellt, daß es die Retentatablaufleitung 8 mit der Leitung 22 verbindet. Bei dieser Stellung der genannten Ventile 19 und 21 wird durch die Förderpumpe 13 das Permeat über die Rückspülleitung 20 in den Permeatraum 6b gefördert und durch die Membran 7 hindurch in den Zulauf- bzw. Retentatraum 6a sowie von dort aus über die Retentatablaufleitung 8 zur Ablaufleitung 23 bzw. über die Leitung 22 in den Fermenter 1. Dabei kann vorübergehend die Förderpumpe 4 abgeschaltet oder aber in der Zulaufleitung 5 ein Absperrventil 24 vorgesehen sein, um während der Rückspülung die Zuführung der Suspension zu der Membranfilteranordnung 6 zu unterbinden.

## Patentansprüche

1. Membrantrennverfahren zur Abtrennung von Flüssigkeiten aus Fermentationssuspensionen, wobei kontinuierlich Suspensionen aus dem Fermenter abgezogen, an einer Porenfiltermembran entlanggeführt und in den Fermenter zurückgeführt wird, **dadurch gekennzeichnet,** daß auf der Permeatseite der Membran ein Unterdruck erzeugt wird, indem das Permeat mittels einer Strahlpumpe im flüssigen Zustand abgesaugt und in eine Kreislaufströmung überführt sowie innerhalb der Kreislaufströmung als Treibmittel der Strahlpumpe zugeführt wird.

2. Verfahren nach Anspruch 1, **dadurch gekennzeichnet,** daß das Permeat während der Kreislaufströmung gekühlt wird.

3. Vorrichtung zur Durchführung des Verfahrens nach Anspruch 1 oder 2 mit einer Membranfilteranordnung mit Einrichtungen für den Zulauf der Suspension und für den Retentatablauf auf der einen Seite sowie Einrichtungen für den Permeatablauf auf der anderen Seite der Membran und einem mit der Permeatablaufleitung verbundenen Sammelbehälter, der mit einer Abführleitung und einer Niveausteuerung für das abzuführende Permeat ausgerüstet ist, **dadurch gekennzeichnet,** daß der Sammelbehälter (12) im Zuge einer Kreislaufleitung (11a bis 11d) in Reihe mit einer Förderpumpe (13) und einer Srahlpumpe (10) angeordnet ist, daß die von der Filteranordnung (6) ausgehende Permeatablaufleitung (9) mit der Strahlpumpe verbunden ist, und daß zwischen der Förderpumpe (13) und der Strahlpumpe (10) ein Dreiwegeventil (19) mit einer in die Permeatablaufleitung (9) mündenden Rückspülleitung (20) vorgesehen und die Retentatablaufleitung (8) über ein Dreiwegeventil (21) mit dem Fermenter (1) für die Suspension verbindbar ist.

## Claims

1. A membrane separating process for separating liquids from fermentation suspensions, wherein suspensions are continuously drawn out of the fermenter, conducted along a porous filter membrane and returned to the fermenter, characterised in that a vacuum is created on the permeate side of the membrane, as the permeate is sucked out in the liquid state by means of a fluid entrainment pump and is transferred into a circulating flow and supplied to the fluid entrainment pump within the flow as a working fluid.

2. A process according to claim 1, characterised in that the permeate is cooled whilst circulating.

3. Apparatus for carrying out the process according to claim 1 or 2 with a membrane filter arrangement with means for supplying the suspension and for draining the residue on the one side and means for draining the permeate on the other side of the membrane, and a collecting vessel, which is connected to the permeate drainage duct and which is equipped with a discharge duct and a level-control device for the permeate to be discharged, characterised in that the collecting vessel (12) is disposed in the wake of a circulating duct (11a to 11d) in series with a feed pump (13) and a fluid entrainment pump (10), in that the permeate drainage duct (9) coming out of the filter arrangement (6) is connected to the fluid entrainment pump, and in that a three-way valve (19) with a reversible flow duct (20) opening into the permeate drainage duct (9) is provided between the feed pump (13) and the fluid entrainment pump (10), and the residue drainage duct (8) is connectable to the fermenter (1) for the suspension via a three-way valve (21).

## Revendications

1. Procédé de séparation à membrane destiné à la séparation de liquides à partir de suspensions de fermentation, les suspensions étant tirées de façon continue à partir du fermenteur, dirigées vers une membrane de filtration poreuse et renvoyée dans le fermenteur, caractérisé en ce qu'il est produit sur le côté à perméat de la membrane une dépression, le perméat étant aspiré à l'état liquide au moyen d'une pompe à jet et envoyé dans un courant à circuit fermé et dirigé également à l'intérieur du courant circulaire sous forme d'agent d'entraînement de la pompe à jet.

2. Procédé selon la revendication 1, caractérisé en ce que le perméat est refroidi pendant son écoulement en circuit fermé.

3. Dispositif pour la mise en œuvre du procédé selon la revendication 1 ou 2, comprenant un dispositif à membrane de filtration avec des dispositifs pour l'amenée de la suspension et l'évacuation du rétentat sur un côté et des dispositifs pour l'évacuation du perméat et un réceptacle collecteur relié à la conduite d'évacuation du perméat sur l'autre côté, réceptacle que est équipé d'une conduite d'évacuation et d'une commande de niveau pour le perméat à évacuer, caractérisé en ce que le réceptacle collecteur (12) est disposé dans une conduite à circuit fermé (11a à 11d) en série avec une pompe de circulation (13) et une pompe à jet (10), en ce que la conduite d'évacuation de perméat (9) qui part de l'agencement de filtration (6) est reliée à la pompe à jet, et en ce qu'il est prévu entre la pompe de circulation (13) et la pompe à jet (10) un distributeur à trois voies (19) avec une conduite à lavage à contre-courant (20) débouchant dans la conduite d'évacuation de perméat (9), et la conduite d'évacuation de rétentat (8) peut être reliée par un distributeur à trois voies (21) au fermenteur (1) destiné à la suspension.